(19) Europäisches Patentamt
European Patent Office
Office européen des brevets

(11) EP 0 577 203 B1

(12) **EUROPEAN PATENT SPECIFICATION**

(45) Date of publication and mention of the grant of the patent: **22.01.1997 Bulletin 1997/04**

(51) Int Cl.6: **C07C 231/10**, C07D 201/04

(21) Application number: **93201841.9**

(22) Date of filing: **25.06.1993**

(54) **Process for the conversion of oximes into the corresponding amides**

Verfahren zur Umwandlung von Oximen zu Amiden

Procédé pour la conversion d'oximes en amides

(84) Designated Contracting States:
**AT BE CH DE DK ES FR GB GR IT LI NL SE**

(30) Priority: **30.06.1992 NL 9201160**

(43) Date of publication of application:
**05.01.1994 Bulletin 1994/01**

(73) Proprietor: **DSM N.V.**
**6411 TE Heerlen (NL)**

(72) Inventors:
- **Thomissen, Petrus Jozef Hubertus**
  **B-3620 Lanaken (BE)**
- **Bosman, Hubertus Johannes Mechtilda**
  **NL-6135 EN Sittard (NL)**

(56) References cited:
**GB-B- 1 342 550**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

Process for the conversion of a ketoxime or aldoxime into a corresponding amide in which a solution of the oxime in an organic solvent is contacted with a strongly acidic catalyst. The invention relates in particular to a process for the conversion of a cyclic ketoxime into the corresponding lactam (cyclic amide).

Such process is disclosed in patent publication GB-B-1342550, which describes an embodiment in which cyclohexanone oxime in dimethyl sulphoxide (DMSO) as solvent is contacted with a strongly acidic catalyst. This catalyst is a sulphonated polystyrene divinyl benzene resin and functions as an ion exchanger.

On an industrial scale the conversion of cyclic ketoximes into the corresponding lactams according to the Beckmann rearrangement - for formation of ε-caprolactam from cyclohexanone oxime - usually takes place in a homogeneous, strongly acidic liquid phase, use being made for example of oleum, optionally dissolved in liquid sulphur dioxide.

The lactam-containing reaction mixture is subsequently neutralized with ammonia water, and the lactam is separated from the resulting ammonium sulphate solution. The next step is recovery of ammonium sulphate from the lactam-free solution by crystallization.

This means that a large amount of ammonium sulphate is produced in the preparation of ε-caprolactam, for instance 1.7-1.9 ton $(NH_4)_2SO_4$ per ton of lactam, if conversion has taken place in oleum. Such an amount of by-product is considered undesirable in view of the increasing difficulties encountered in selling ammonium sulphate and the attendant environmental problems, and for this reason methods have been sought for quite some time already to effect the conversion of oximes into lactams without ammonium sulphate by-product being obtained.

It has been suggested to carry out the conversion at high temperatures in the gas phase in the presence of solid acid catalysts, such as boron oxide on silica as described in DE-B-2053065, but this method is technically and economically less attractive because the process flows - gases in place of liquids - occupy a large volume, with as a consequence that the equipment costs and upgrading costs will be high compared with a liquid phase process. In addition, the high temperature of the gas phase process does not seem advantageous for the quality of the lactam to be produced.

In the patent publication referred to, GB-B-1342550, it was suggested to have the conversion of the oxime into the corresponding lactam take place under the influence of a strongly acidic ion exchanger in the $H^+$ form, the ion exchanger being contacted with an oxime dissolved in a solvent. In contrast, the conversion of oximes into lactams in a homogeneous environment of oleum, optionally dissolved in liquid sulphur dioxide, the lactam formed is released by neutralization of the acid with ammonia. In case of application of a strongly acidic ion exchanger such a neutralization is not necessary because no oleum is used. However, the lactam selectivity and oxime conversion belonging to this known method described in GB-B-1342550 are not high enough for this process to compete with the above-mentioned rearrangement method using oleum.

The present invention aims to provide an improved embodiment, with a higher lactam selectivity and a higher lactam productivity, for the rearrangement of cyclic ketoximes into the corresponding lactams using ion exchangers.

This aim is achieved by contacting a solution of an oxime in a solvent with a heterogeneous, strongly acidic catalyst, the heterogeneous catalyst being a carrier with sulphonated benzene rings and the sulphonated benzene rings also carrying an electron-withdrawing group.

It has been found that the presence of electron-withdrawing groups on the sulphonated benzene ring has a positive effect on the lactam selectivity and the lactam productivity.

Examples of ketoximes or aldoximes that can be treated according to the present invention include unsaturated and saturated, substituted or unsubstituted aliphatic ketoximes or aldoximes or cyclic ketoximes with 2-30 carbon atoms, such as acetone oxime, acetaldoxime, benzaldoxime, propanaldoxime, butanaldoxime, butanone oxime, butene-1-onoxime, cyclopropanone oxime, cyclohexanone oxime, cyclooctanone oxime, cyclododecanone oxime, cyclopentenone oxime, cyclododecanone oxime, 2-phenyl cyclohexanone oxime, cyclohexenone oxime.

As raw material for the process according to the invention use is preferably made of a cyclic ketoxime. Preferably, the cyclic ketoxime has 5-12, in particular 6-12 carbon atoms. Examples of extremely suitable cyclic ketoximes are cyclohexanone oxime, cyclooctanone oxime and cyclododecanone oxime.

This specification and the examples will be based in particular on cyclohexanone oxime, for in this embodiment the commercially interesting ε-caprolactam is formed.

Possible solvents may be either inorganic or organic or mixtures of both.

The ketoxime is preferably dissolved in an organic solvent. Possible organic solvents described in GB-B-1342550 are toluene, dimethylformamide and mixtures of acetic acid anhydride, toluene and cyclohexane. An organic sulphoxide of the general formula $R^1SOR^2$, where $R^1$ and $R^2$ represent $C_1$-$C_5$ alkyl or $C_6$-$C_{12}$ aryl groups, is a very suitable solvent. Of this group of organic sulphoxides, preference is given to dimethyl sulphoxide (DMSO) because this substance is prepared on an industrial scale and can be obtained at a reasonable price.

Disregarding the price, there are also other organic sulphoxides that may be used, such as diethyl sulphoxide, dibutyl sulphoxide, divinyl sulphoxide and diphenyl sulphoxide.

The oxime concentration in the organic solvent as a rule is between 0.1 wt.% and 40 wt.%, and preferably between 0.5 wt.% and 30 wt.% and most preferably between 1 and 20 wt.%..

The catalyst consists of a carrier combined with sulphonated benzene rings, the sulphonated benzene rings being modified with an electron-withdrawing group. The catalyst is capable of ion exchange. The ion-exchanging capacity is expressed in meq of $H^+$ per gramme of dry catalyst.

The catalyst according to the invention customarily has a capacity between 0.1 meq $H^+$ and 6 meq $H^+$ per gramme of dry catalyst.

The modified benzene rings can be described by the following general formula:

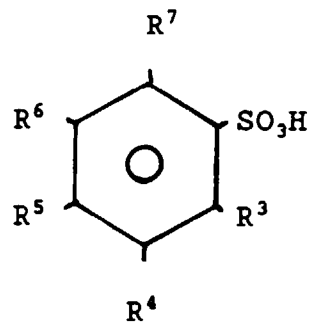

(I)

where $R^3$ to $R^7$ independently represent one or more electron-withdrawing groups, to be chosen from a nitro ($-NO_2$), halogen (-Cl, -Br, -F, -I), a sulphonic acid ($-SO_3H$), a hydroxy, an alkoxy or a cyano ($-C{\equiv}N$) group, the remaining groups representing hydrogen, an alkyl or an aryl alkyl and where at least one group is represented by

$$-R\text{-}P \quad \text{or} \quad -P \qquad \text{(II),(III)}$$

wherein R can be a substituted or unsubstituted $C_1$-$C_5$ alkyl or a substituted or unsubstituted $C_6$-$C_{12}$ aryl or arylalkyl and wherein P is part of an organic or anorganic carrier.

Preferably, the electron-withdrawing group is a -Cl, -F, $-SO_3H$ or an $-NO_2$ group. R in formula II can also have electron withdrawing capacity by introducing electron withdrawing groups on R.

Preferably, a benzene ring carries 1-3 electron-withdrawing groups, and in particular 1-2 electron-withdrawing groups.

The carrier is a material that is solid under the process conditions and that is not or hardly dissolved in the solvent used. The carrier may be of organic or inorganic origins.

Examples of organic carrier P are (lineair) carbon polymers such as polyethylene, polypropylene and butadiene. Usually one or two -P or -R-P groups are present on the benzene ring. Examples of suitable resins are polystyrene (one -P group wherein P is polyethylene) poly-divinylbenzene (two -P groups wherein P is polyethylene) and mixtures of these resins in which benzene rings with one and benzene rings with two -P groups are present, such as polystyrene divinylbenzene resin. These resins are commercially available and can be easily sulphonated with e.g. oleum.

R in formula II can be a methyl, ethyl, propyl-or fenylenegroup. R can also be another sulphonated benzene ring connected with the benzene ring of formula I via a formylgroup as described in DE-A-3544210.

A preferred embodiment according to the invention is a sulphonated polystyrene divinyl benzene resin in which the sulphonated benzene ring also carries an electron-withdrawing group. Such an ion exchanger with chlorine or bromine as electron-withdrawing groups is described in US-A-4,269,943. If this sulphonated polystyrene divinyl benzene resin, modified with electron-withdrawing groups, is used as catalyst, the ion exchanger unexpectedly appears to have a longer stability than the state-of-the-art ion exchanger during rearrangement of oximes into lactams. An added advantage of the increased stability of the ion exchanger according to the invention is that it is possible to work at higher temperatures, so that productivity is increased further. Productivity as used here stands for an amount of lactam obtained per active catalyst site and per unit of time. The amount of active catalyst sites of a gram catalyst is expressed in eq $H^+$ per gram catalyst and is the same as the earlier mentioned capacity.

Examples of inorganic carriers are: carbon and metal oxides with free OH groups, such as $SiO_2$, $Al_2O_3$, ZnO, $TiO_2$ and MgO. The bond between the sulphonated benzene ring and the carrier may be a chemical one (direct or, for example, via an alkyl group). An example of an inorganic carrier combined with sulphonated benzene rings is $SiO_2$ bound to the sulphonated benzene ring via an alkyl group:

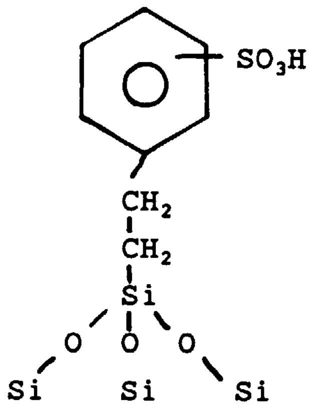

(IV)

For a reasonable reaction velocity a temperature of 75-200°C, and preferably from 110°C to 150°C, is desirable.

As embodiment for a process according to the invention a continuous process is preferred over batchwise production, for in a continuous process a higher oxime conversion and lactam yield are achieved. Preferably, a fixed bed reactor is used, but a continuously stirred tank reactor as embodiment for the reactor is also possible.

The invention will be elucidated on the basis of the following examples, without being limited thereto.

In the examples a number of terms are used which, for the sake of clarity, are explained below:

$$\text{Oxime conversion (\%)} = \frac{\text{mol of reacted oxime}}{\text{mol of original oxime}} * 100\%$$

$$\text{Lactam selectivity (\%)} = \frac{\text{mol of lactam obtained}}{\text{mol of reacted oxime}} * 100\%$$

$$\text{Anone selectivity (\%)} = \frac{\text{mol of anone obtained}}{\text{mol of reacted oxime}} * 100\%$$

$$\text{Lactam yield (\%)} = \text{lactam selectivity} * \text{oxime conversion}/100\%$$

The ion exchanger capacity is expressed in meq $H^+$ per gramme of dry ion exchanger.

In the examples use is made of commercially available polystyrene divinyl benzene resin from Rohm and Haas.

The ion exchangers used are:

| | |
|---|---|
| Amberlyst 15 (*): | sulphonated polystyrene divinyl resin as reference material |
| Amberlyst 17 : | sulphonated polystyrene divinyl resin modified with chlorine |
| Amberlyst 35 : | sulphonated polystyrene divinyl resin modified with a second sulphonic acid group on the benzene ring. |

**Example I**

Ion exchanger Amberlyst 17, with a capacity of 3.26 meq $H^+$ per gram of dry catalyst, was pretreated by successive washes with 5 n HCl, water and DMSO.

The Initial cyclohexanone oxime concentration was 5 wt.% in DMSO. The reactor contents consisted of 0.25 1 of this mixture and an amount of ion exchanger as stated in Table 1. The temperature was 115°C, the stirring speed 2000 rpm and the reaction time 3 hours. The ε-caprolactam productivity proved to be 0.76 mol of ε-caprolactam per equivalent of H+ per hour. Some other results are summarized in Table 1.

(*) Amberlyst is a tradename of Rohm and Haas.

**Example II**

Example I was repeated, now with Amberlyst 35, with a capacity of 5.55 meq of $H^+$ per gram of dry catalyst. The results are summarized in Table 1.

**Example III**

At room temperature 5 times 5 gram of dry Amberlyst 15 ion exchanger, which had previously been vacuum-dried at 60°C, were added (at 15 minutes intervals) to a mixture of 41.5 gram of 65% $HNO_3$ and 73.6 gram of $H_2SO_4$ (hereafter to be referred to as nitrating acid) in a 250 ml round-bottom flask. The reacting mixture was then heated to 95°C, following which the mixture was kept at this temperature for 10 minutes. Subsequently, the mixture was slowly cooled down to room temperature during 2 hours, after which the ion exchanger was filtered off from the nitrating acid and washed five times with demineralized water. The reaction of the wash water then still is mildly acidic. (pH = 6).

The resulting exchanger had a capacity of 2.36 meq of $H^+$ per gram of dry catalyst.

**Example IV**

Example I was repeated, but now the ion exchanger obtained in Example III was used. The results are summarized in Table 1.

**Example V**

Example I was repeated, now at a temperature of 150°C. The ratio between mol of cyclohexanone oxime and equivalents of $H^+$ was 4.1 at t=0. The ε-caprolactam productivity proved to be 1.07 mol of lactam per equivalent of $H^+$ per hour. The results are summerized in Table 1.

**Example VI**

In a stirred reactor 1.5 kg of a mixture as obtained in Example I was contacted for 2 minutes with 10 g of water that contained phosphoric acid and had a pH of 1.8, the temperature being 100°C. 100% of the cyclohexanone oxime was hydrolyzed into cyclohexanone and hydroxylamine. By means of a batch distillation the water was subsequently distilled off at a pressure of 50 mm Hg. The distillation was continued at reduced pressure (15 mm Hg), cyclohexanone and 16.1 g ε-caprolactam being recovered from the DMSO solution.

**Comparative Experiment A**

Example I was repeated, now use being made of Amberlyst 15, with a capacity of 4.11 meq of $H^+$ per gram of dry catalyst. The ε-caprolactam productivity was 0.25 mol of lactam per equivalent of $H^+$ per hour. Some other results are summarized in Table 1.

**Table 1**

| Experiment | mol oxime/eq $H^+$ at t=0 | oxime conv. (%) | selectivity[2)] lactam (%) | anone (%) | lactam yield (%) | lactam productivity[1)] |
|---|---|---|---|---|---|---|
| I | 3.3 | 74.4 | 88.1 | 11.9 | 65.5 | 0.76 |
| II | 3.7 | 46.3 | 81.9 | 18.1 | 37.9 | 0.53 |
| IV | 4.0 | 55.1 | 87.5 | 12.5 | 48.2 | 0.65 |
| V[3)] | 4.1 | 93.1 | 83.9 | 16.1 | 78.1 | 1.07 |
| A | 4.0 | 54.6 | 34.2 | 13.8 | 18.7 | 0.25 |

Catalyst activity at t = 3 hours and 115°C.

1) ε-caprolactam (lactam) productivity expressed in mol of ε-caprolactam per equivalents of $H^+$ per hour.

2) anone = cyclohexanone, lactam = ε-caprolactam

3) T = 150°C

## Claims

1. Process for the conversion of a ketoxime or an aldoxime into a corresponding amide in which a solution of the oxime in a solvent is contacted with a heterogeneous, strongly acidic catalyst, characterized in that the heterogenous catalyst is a carrier with sulphonated benzene rings, the sulphonated benzene rings also carrying an electron-withdrawing group.

2. Process according to claim 1, characterized in that the benzene rings are described by the following general formula

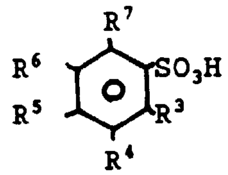

where $R^3$ to $R^7$ independently represent one or more electron withdrawing groups, the remaining groups representing hydrogen, an alkyl or an arylalkyl and where at least one group of $R^3$ to $R^7$ is represented by

-R-P or -P

wherein R represents a substituted or unsubstituted $C_1$-$C_5$ alkyl or a substituted or unsubstituted $C_6$-$C_{12}$ aryl or arylalkyl and wherein P is part of an organic or anorganic carrier.

3. Process according to claim 1, characterized in that the carrier is of organic origins.

4. Process according to claim 3, characterized in that part of the benzene rings have one and part of the benzene rings have two -P groups and that P represents a lineair carbon chain to form a sulphonated polystyrene divinyl benzene resin.

5. Process according to any one of claims 1-4, characterized in that the electron-withdrawing group may be a nitro ($-NO_2$), a halogen (-Cl, -Br, -F, -I) or a sulphonic acid ($-SO_3H$) group.

6. Process according to any one of claims 1-5, characterized in that the solvent is an organic solvent.

7. Process according to claim 6, characterized in that the organic solvent is an organic sulphoxide of the general formula $R^1SOR^2$, in which $R^1$ and $R^2$ represent $C_1$-$C_5$ alkyl or $C_6$-$C_{12}$ aryl groups.

8. Process according to claim 7, characterized in that the organic sulphoxide is dimethyl sulphoxide (DMSO).

9. Process according to any one of claims 1-8, characterized in that the conversion takes place at a temperature above 75°C.

10. Process according to any one of claims 1-9, characterized in that the oxime is a cyclic ketoxime.

11. Process according to claim 10, characterized in that the cyclic ketoxime has 6-12 carbon atoms.

12. Process according to claim 10, characterized in that the cyclic ketoxime is a cyclohexanone oxime.

## Patentansprüche

1. Verfahren zur Überführung eines Ketoxims oder eines Aldoxims in ein entsprechendes Amid, bei welchem eine Lösung des Oxims in einem Lösungsmittel mit einem heterogenen, stark sauren Katalysator in Berührung gebracht wird, dadurch gekennzeichnet, daß der heterogene Katalysator ein Träger mit sulfonierten Benzolringen ist, wobei die sulfonierten Benzolringe auch einen Elektronenfänger-Gruppe tragen.

**2.** Verfahren nach Anspruch 1, dadurch gekennzeichnet, daß die Benzolringe durch die folgende allgemeine Formel beschrieben werden:

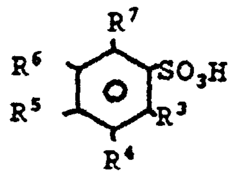

,

worin $R^3$ bis $R^7$ unabhängig eine oder mehrere Elektronenfänger-Gruppen bedeuten, wobei die verbleibenden Gruppen Wasserstoff, ein Alkyl oder ein Arylalkyl sind, und worin zumindest eine der Gruppen $R^3$ bis $R^7$ dargestellt wird durch

-R-P oder -P,

worin R ein substituiertes oder unsubstituiertes $C_1$-$C_5$-Alkyl oder ein substituiertes oder unsubstituiertes $C_6$-$C_{12}$-Aryl oder -Arylalkyl bedeutet, und worin P ein Teil eines organischen oder anorganischen Trägers ist.

**3.** Verfahren nach Anspruch 1, dadurch gekennzeichnet, daß der Träger organischen Ursprungs ist.

**4.** Verfahren nach Anspruch 3, dadurch gekennzeichnet, daß ein Teil der Benzolringe eine und ein Teil der Benzolringe zwei Gruppen -P aufweist, und daß P eine lineare Kohlenstoffkette bedeutet, wobei ein sulfoniertes Polystyroldivinylbenzolharz gebildet wird.

**5.** Verfahren nach einem der Ansprüche 1 bis 4, dadurch gekennzeichnet, daß die Elektronenfänger-Gruppe eine Nitro- (-$NO_2$), eine Halogen- (-Cl, -Br, -F, -I) oder eine Sulfonsäure- (-$SO_3H$) Gruppe sein kann.

**6.** Verfahren nach einem der Ansprüche 1 bis 5, dadurch gekennzeichnet, daß das Lösungsmittel ein organisches Lösungsmittel ist.

**7.** Verfahren nach Anspruch 6, dadurch gekennzeichnet, daß das organische Lösungsmittel ein organisches Sulfoxid der allgemeinen Formel $R^1SOR^2$ ist, worin $R^1$ und $R^2$ $C_1$-$C_5$-Alkyl- oder $C_6$-$C_{12}$-Aryl-Gruppen bedeuten.

**8.** Verfahren nach Anspruch 7, dadurch gekennzeichnet, daß das organische Sulfoxid Dimethylsulfoxid (DMSO) ist.

**9.** Verfahren nach einem der Ansprüche 1 bis 8, dadurch gekennzeichnet, daß die Überführung bei einer Temperatur von mehr als 75°C stattfindet.

**10.** Verfahren nach einem der Ansprüche 1 bis 9, dadurch gekennzeichnet, daß das Oxim ein cyclisches Ketoxim ist.

**11.** Verfahren nach Anspruch 10, dadurch gekennzeichnet, daß das cyclische Ketoxim 6 bis 12 Kohlenstoffatome aufweist.

**12.** Verfahren nach Anspruch 10, dadurch gekennzeichnet, daß das cyclische Ketoxim ein Cyclohexanonoxim ist.

## Revendications

**1.** Procédé de conversion d'un cétoxime ou d'un aldoxime en amide correspondant dans lequel on met en contact une solution de l'oxime dans un solvant avec un catalyseur hétérogène fortement acide, caractérisé en ce que le catalyseur hétérogène est un support avec des noyaux benzène sulfoné, les noyaux de benzène sulfoné supportant également un groupe de retenue d'électrons.

**2.** Procédé selon la revendication 1, caractérisé en ce que les noyaux de benzène sont décrits par la formule générale suivante :

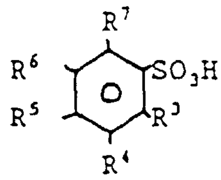

dans laquelle $R^3$ à $R^7$ représentent indépendamment un ou plusieurs groupes de retenue d'électrons, les groupes restants représentant l'hydrogène, un alkyle ou un arylalkyle et dans laquelle au moins un groupe parmi $R^3$ à $R^7$ est représenté par

-R-P ou -P

R représentant un alkyle en $C_{1-5}$ substitué ou non substitué ou un aryle ou arylalkyle en $C_{6-12}$ substitué ou non substitué et P étant une partie d'un support organique ou minéral.

**3.** Procédé selon la revendication 1, caractérisé en ce que le support est d'origine organique.

**4.** Procédé selon la revendication 3, caractérisé en ce qu'une partie des noyaux benzène ont un groupe -P et une partie des noyaux benzène ont deux groupes -P et en ce que P représente une chaîne carbone linéaire pour former une résine polystyrène-divinylbenzène sulfonée.

**5.** Procédé selon l'une quelconque des revendications 1 à 4, caractérisé en ce que le groupe de retenue d'électrons peut être un groupe nitro ($-NO_2$), halogène (-Cl, -Br, -F, -I) ou acide sulfonique ($-SO_3H$).

**6.** Procédé selon l'une quelconque des revendications 1 à 5, caractérisé en ce que le solvant est un solvant organique.

**7.** Procédé selon la revendication 6, caractérisé en ce que le solvant organique est un sulfoxyde organique de formule générale $R^1SOR^2$ dans laquelle $R^1$ et $R^2$ représentent des groupes alkyle en $C_{1-5}$ ou aryle en $C_{6-12}$.

**8.** Procédé selon la revendication 7, caractérisé en ce que le sulfoxyde organique est le diméthylsulfoxyde (DMSO).

**9.** Procédé selon l'une quelconque des revendications 1 à 8, caractérisé en ce que la conversion a lieu à une température au dessus de 75°C.

**10.** Procédé selon l'une quelconque des revendications 1 à 9, caractérisé en ce que l'oxime est un cétoxime cyclique.

**11.** Procédé selon la revendication 10, caractérisé en ce que le cétoxime cyclique a 6 à 12 atomes de carbone.

**12.** Procédé selon la revendication 10, caractérisé en ce que le cétoxime cyclique est un cyclohexanoneoxime.